# EUROPÄISCHE PATENTANMELDUNG

(11) **EP 1 319 367 A2**
(43) Veröffentlichungstag der Anmeldung: **18.06.2003**
(21) Anmeldenummer: 02024511.4
(22) Anmeldetag: 30.10.2002
(51) Int. Cl.: A61B 5/15

(54) **Reservoir für Blutentnahme**

(30) Priorität: 11.12.2001 DE 10160703
(71) Anmelder: Smiths Medical Deutschland GmbH, 85614 Kirchseeon (DE)
(72) Erfinder: Weber, Jörg, 83533 Edling (DE); Pfandl, Peter, 85540 Haar (DE)
(74) Vertreter: von Bülow, Tam, Dr.

(57) **Zusammenfassung**

Das Reservoir für die Blutentnahme hat ein Gehäuse (1) und einen darin verschieblichen Kolben (7), wodurch im Inneren des Gehäuses eine Reservoir-Kammer (16) variabler Größe gebildet wird. Der Kolben ist mit einem Betätigungsorgan (11) gekoppelt. Weiter ist der Kolben (7) durch eine erste Feder (14) in eine Schließstellung gedrückt, in welcher die Reservoir-Kammer (16) minimales Volumen hat. Das Betätigungsorgan (11) und der Kolben (7) sind über eine zweite Feder (10) lose miteinander gekoppelt, wobei der Federweg dieser Feder (10) kleiner ist als der Verschiebeweg (13) des Betätigungsorganes, so daß am Ende der Bewegung eines Zwangskopplung zwischen dem Kolben und dem Betätigungsorgan (11) erfolgt.

## Beschreibung

Die Erfindung bezieht sich auf ein Reservoir für eine Blutentnahme gemäß dem Oberbegriff des Patentanspruches 1. Ein derartiges Reservoir ist beispielsweise aus der DE 297 13 743 U1 bekannt.

Patienten, denen ein Infusionsschlauch oder ein Katheter in eine Vene oder Arterie eingesetzt ist, muß des öfteren für Untersuchungszwecke eine Blutprobe entnommen werden, was zweckmäßigerweise an einer von außen zugänglichen Stelle des Infusionsschlauches erfolgt. Hierzu ist eine Blutentnahmestelle in dem Infusionssystem vorgesehen. Bevor die Blutentnahme stattfinden kann, muß dafür gesorgt werden, daß sich im Bereich der Entnahmestelle nur unverdünntes Blut des Patienten und keine Anteile von Infusionslösungen oder Verdünnungsmitteln befinden.

Die WO 88/01846 sieht daher zwei Entnahmestellen vor. Die näher zum Patienten liegende, vordere Entnahmestelle dient der eigentlichen Entnahme der Blutprobe, die weiter vom Patienten entfernt liegende hintere Entnahmestelle dient dazu, vorübergehend Infusionslösungen aus dem System zu entfernen, damit an der vorderen Entnahmestelle nur noch unverdünntes Blut vorhanden ist. Diese zweite Entnahmestelle hat also die Funktion eines Reservoirs. Nach der Blutentnahme an der vorderen Entnahmestelle wird dann die in dem Reservoir zwischengespeicherte Menge wieder dem Patienten infudiert.

Die DE 297 13 743 U1 zeigt ein solches Reservoir mit einer Kammer, die an einer Verzweigung eines Infusionsschlauches angeschlossen ist. Ein Kolben ist in dieser Kammer verschieblich und kann durch Zurückziehen Blut und/oder Infusionslösung in die Kammer saugen. Eine Kolbenstange des Kolbens ist mit einem Griff befestigt, wobei zwischen diesem Griff und einer Abstützung in der Kammer eine Feder vorgesehen ist, die den Griff und damit den Kolben in einer Richtung zur Blutentnahme drückt, so daß das Ansaugen von Blut oder Infusionslösung in die Kammer weitestgehend automatisch erfolgt. Der Benutzer muß lediglich eine Verriegelung am Griff lösen. Ähnliche Blutentnahme-Vorrichtungen mit einer Feder, die ein selbsttätiges Ansaugen bewirkt, sind auch der DE 94 19 737 U1 und der DE 40 19 197 C2 zu entnehmen. Generell wird für Blutentnahmestellen dieser Art, d.h. Reservoirs, auch noch auf die DE 42 20 301 C1 und die EP 0 301 913 A2 verwiesen.

Ein Vorteil der aufgrund Federkraft selbstansaugenden Blutentnahme-Vorrichtungen liegt in der Einhand-Bedienung. Dies begründet aber auch eine Gefahr, da durch den beim Ansaugen entstehenden Unterdruck Gasbläschen gebildet werden können, die beim späteren Reinfudieren in den Kraislauf des Patienten gelangen. Dies ist besonders dann der Fall, wenn der Betätigungsgriff unfreiwillig entriegelt wird und das Ansaugen stattfindet, wenn bestimmte Ventile in den Infusionsleitungen geschlossen sind.

Aufgabe der Erfindung ist es, das Reservoir der eingangs genannten Art dahingehend zu verbessern, daß sie bei einfacher Bedienung eine höhere Sicherheit bietet.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Bei der Erfindung wird ebenfalls eine Feder verwendet, die jedoch zum Leeren des Reservoirs und nicht zum Ansaugen dient. Dies erhöht die Sicherheit, da ein ungewolltes Ansaugen nicht mehr möglich ist, vereinfacht dafür das Entleeren des Reservoirs.

Zur weiteren Erhöhung der Sicherheit ist der Kolben über eine weitere Feder vom Betätigungsorgan entkoppelt. Über die Härte dieser weiteren Feder läßt sich damit der maximal mögliche Unterdruck ausgleichen, der entstehen kann, wenn angesaugt wird und die Ventile geschlossen sind.

Nach einer Weiterbildung der Erfindung liegt das Reservoir "in Linie" mit einem Infusionsschlauch, hat also einen getrennten Einlaß und Auslaß, die durch ein Rohr miteinander verbunden sind. Vorzugsweise ist dieses Rohr starr und ist zentral im Gehäuse angeordnet, so daß der verschiebliche Kolben längs diesem Rohr geführt wird. Es sei allerdings darauf hingewiesen, daß die Blutentnahme-Vorrichtung auch in bekannter Weise seitlich an eine Infusionsleitung angeschlossen werden kann, beispielsweise gemäß der DE 42 20 301 C1

Im folgenden wird die Erfindung anhand eines Ausführungsbeispieles im Zusammenhang mit der Zeichnung ausführlicher erläutert.

Die einzige Figur zeigt einen Querschnitt des Reservoirs.

Das Reservoir besitzt ein im wesentlichen zylindrisches Gehäuse 1, das an beiden axialen Enden Anschlüsse 2 und 3 hat, an denen Schlauchabschnitte 4 und 5 anschließbar sind. Zentral im Inneren des Gehäuses ist ein starres Rohr 6 angeordnet, das am patientenfernen Anschluß 2 unmittelbar in den Schlauch 4 mündet, während es am patientenseitigen Ende nahe dem Anschluß 3 in eine Kammer 16 mündet. Im Inneren des Gehäuses ist ein verschieblicher Kolben 7 angeordnet, der an dem starren Rohr 6 geführt ist und seitlich dichtend an der Innenwand des Gehäuses 1 anliegt.

Der Kolben 7 ist mit einem ersten Mitnehmer 8 verbunden, der hier etwa rechteckigen Querschnitt hat und eine untere Öffnung 8.1 für den Durchtritt des Rohres 6 sowie eine obere Öffnung 8.2 für den Durchtritt eines zweiten Mitnehmers 9. Die untere Wand 8.3 des ersten Mitnehmers 8 ist mit der Oberseite des Kolbens 7 fest verbunden, beispielsweise verklebt.

Ein zweiter Mitnehmer 9, der im Querschnitt etwa doppel-Tförmig ist, ragt mit einem axialen Schenkel 9.1 durch die Öffnung 8.2 des ersten Mitnehmers in dessen Innenraum, wo er an einem ersten Querschenkel 9.2 endet, an dem sich eine zweite Feder 10 abstützt. Das andere Ende der zweiten Feder 10 stützt sich an der oberen Innenwand 8.4 des ersten Mitnehmers ab. Ein zweiter Querschenkel 9.3 des zweiten Mitnehmers ist außerhalb des ersten Mitnehmers. An ihm stützt sich eine erste Feder 14 ab, deren anderes Ende an einer Federabstützung 17 anliegt, die am Ende des Gehäuses 1 nahe dem Anschluß 2 angeordnet ist. Der zweite Mitnehmer 9 weist einen Stift 12 auf, der durch einen Schlitz 13 im Gehäuse 1 nach außen ragt und dort mit einem Betätigungsorgan, wie z.B. einem Ring 11 oder einem Knopf, verbunden ist. Am oberen, d.h. dem Anschluß 2 nahegelegenen Ende des Schlitzes 13 ist eine Bajonett-Verriegelung 13.1 vorgesehen, in welcher der Stift einrasten kann.

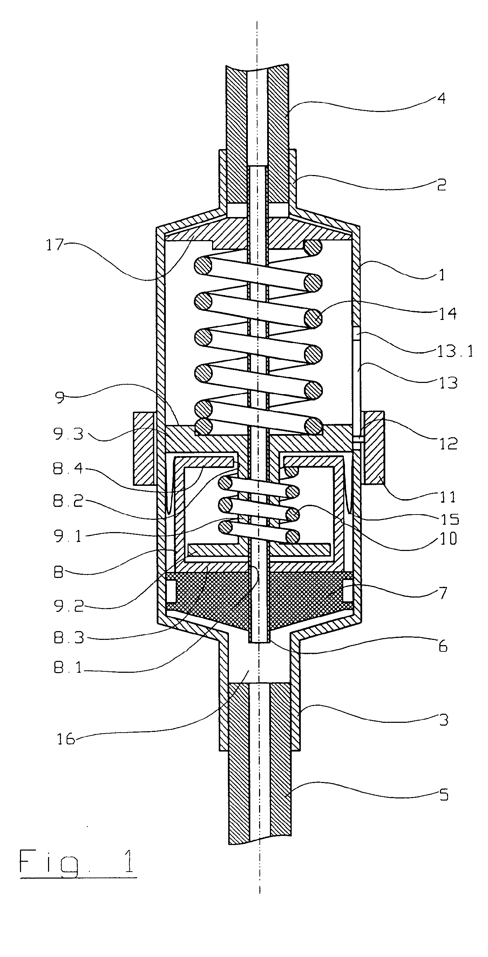

Im folgenden wird die Arbeitsweise der Vorrichtung beschrieben. Ausgehend von der in Fig. 1 dargestellten "Ruhestellung" wird das Betätigungsorgan 11 von Hand nach oben, d.h. in Richtung zum Anschluß 2 gezogen. Dabei wird der zweite Mitnehmer 9 gegen die Kraft der Feder 14 verschoben und der Quersteg 9.2 des zweiten Mitnehmers nimmt über die zweite Feder 10 den ersten Mitnehmer 8 mit, der seinerseits den Kolben 7 dann verschiebt, so daß das Volumen der Kammer 16 vergrößert wird. Aus dem Schlauch 5 wird dadurch Flüssigkeit in die Kammer 16 gesaugt und dort zwischengespeichert. Erreicht der Stift 12 das obere Ende des Schlitzes 13, so kann er durch Bewegung in Umfangsrichtung in die Bajonettrasterung 13.1 eingerastet werden und die Vorrichtung speichert ein entsprechendes Volumen in der maximal geöffneten Kammer 16.

Wie bei solchen Blutentnahme-Vorrichtungen üblich, wird vor Beginn des Ansaugens eine nicht dargestelltes Ventil im Schlauch 4 geschlossen, so daß nur Flüssigkeit aus dem Schlauch 5 angesaugt wird.

Das Betätigungsorgan 11 und der zweite Mitnehmer 9 sind gegenüber dem Kolben 7 durch die zweite Feder 10 entkoppelt. Der Kolben folgt also der Bewegung des zweiten Mitnehmers nicht unmittelbar sondern entsprechend der jeweils gewählten Härte der zweiten Feder 10 nur in solchem Maße, daß der Unterdruck in der Kammer 16 ein vorbestimmtes Maß nicht übersteigt.

Das Reinfudieren der Flüssigkeit und damit das Entleeren der Kammer erfolgt in umgekehrter Reihenfolge. Wird der Stift 12 aus der Bajonett-Rasterung 13.1 gelöst, so drückt die erste Feder 14 den zweiten Mitnehmer nach unten und dieser wiederum über den Steg 8.4 unmittelbar den ersten Mitnehmer 8, der den Kolben 7 nach unten drückt. In dieser Richtung sind die beiden Mitnehmer direkt gekoppelt, da für das Reinfudieren keine Entkopplung benötigt wird.

Abschließend sei noch darauf hingewiesen, daß zwischen einem der beiden Mitnehmer 8 und 9 und der Innenwand des Gehäuses 1 eine Abdichtung in Form eines Rollschlauches 15 vorgesehen sein kann, der angrenzend an den Schlitz 13 in Richtung zum patientenseitigen Anschluß 3 angeordnet ist und damit verhindert, daß durch den Schlitz 13 Verunreinigungen in den Teil des Gehäuses 1 gelangen können, die mit der Kammer 16 in Verbindung stehen. Selbstverständlich geht der Rollschlauch rings um den gesamten ersten Mitnehmer 8.

Weiter sei noch erwähnt, daß der Anschluß 2 zweckmäßigerweise gegenüber dem Schlauch 4 als fester Anschluß ausgebildet ist, während der Anschluß 3 gegenüber dem Schlauch als lösbarer Anschluß dient, beispielsweise durch eine Luer-Kupplung oder eine Verschraubung.

Der Abstand der Querstege 8.4 und 9.2 sowie die Dicke der Feder 10 im vollständig zusammengedrückten Zustand sind kleiner als die Länge des Schlitzes 13, womit sichergestellt ist, daß sich der Kolben 7 stets aus seiner vorderen Endstellung lösen kann, selbst wenn er dort angeklebt ist, beispielsweise durch Blutreste. In der letzten Phase der Bewegung des Mitnehmers 9.3 ist somit eine Zwangskopplung vorgesehen, da der Federweg der Feder 10 vollständig durchlaufen ist und diese dann keine Federwirkung mehr zeigt, sondern als starres Teil wirkt.

## Patentansprüche

1. Reservoir für eine Blutentnahme mit einem Gehäuse (1), in welchem ein Kolben (7) über ein Betätigungsorgan (11) verschieblich ist und eine Bewegungsrichtung des Kolbens (7) durch eine Feder (14) unterstützt ist, **dadurch gekennzeichnet,**
**daß** die Feder (14) den Kolben (7) in eine Schließstellung drückt, in welcher die Kammer (16) des Gehäuses (1) ihr minimales Volumen hat.

2. Reservoir nach Anspruch 1, **dadurch gekennzeichnet, daß** der Kolben (7) und das Betätigungsorgan (11) durch eine zweite Feder (10) miteinander gekoppelt sind.

3. Reservoir nach Anspruch 2, **dadurch gekennzeichnet, daß** der Kolben (7) mit einem ersten Mitnehmer (8) fest verbunden ist,
daß das Betätigungsorgan (11) mit einem zweiten Mitnehmer (9) fest verbunden ist und
daß die beiden Mitnehmer (8 und 9) über die zweite Feder (10) miteinander derart gekoppelt sind, daß bei einer Öffnungsbewegung des Kolbens (7) der zweite Mitnehmer (9) über die zweite Feder (10) den ersten Mitnehmer und damit den Kolben bewegt.

4. Reservoir nach Anspruch 3, **dadurch gekennzeichnet, daß** der Federweg der zweiten Feder kleiner ist als der maximal mögliche Verschiebeweg des zweiten Mitnehmers (9), so daß am Ende des Federweges der zweiten Feder (10) eine Zwangskopplung zwischen dem Betätigungsorgan (11) und dem Kolben (7) erfolgt.

5. Reservoir nach einem der Ansprüche 1 bis 4, **dadurch gekennzeichnet,**
**daß** das Gehäuse (1) in Axialrichtung einen ersten Anschluß (2) und einen zweiten Anschluß (3) aufweist und daß ein starres Rohr (6) zentral zur Achse des Gehäuses (1) vorhanden ist, dessen eines Ende mit dem Anschluß (2) verbunden ist und dessen anderes Ende in die Kammer (16) mündet.

6. Reservoir nach einem der Ansprüche 1 bis 5, **dadurch gekennzeichnet,**
**daß** das Betätigungsorgan (11) über einen Stift (12), der einen Längsschlitz (13) des Gehäuses (1) durchdringt, mit dem zweiten Mitnehmer (9) verbunden ist.

7. Reservoir nach Anspruch 6, **dadurch gekennzeichnet, daß** der Schlitz (13) eine Bajonett-Rasterung (13.1) für den Stift (12) aufweist.

8. Reservoir nach einem der Ansprüche 1 bis 7, **dadurch gekennzeichnet,**
**daß** wirkungsmäßig zwischen der Innenwand des Gehäuses (1) und dem Kolben (7) eine Rolldichtung (15) vorgesehen ist.

9. Reservoir nach Anspruch 8, **dadurch gekennzeichnet, daß** die Rolldichtung (15) einerseits an der Innenwand des Gehäuses (1) und andererseits am ersten oder am zweiten Mitnehmer (8 oder 9) befestigt ist.
